# EUROPÄISCHE PATENTANMELDUNG

(11) **EP 1 958 598 A1**
(43) Veröffentlichungstag der Anmeldung: **20.08.2008**
(21) Anmeldenummer: 07003351.9
(22) Anmeldetag: 16.02.2007
(51) Int. Cl.: A61F 2/90

(54) **Wachstumsfähige, rohrförmige Stützprothese**

(71) Anmelder: Universität Zürich, 8006 Zürich (CH)
(72) Erfinder: Die Erfindernennung liegt noch nicht vor
(74) Vertreter: Grünecker, Kinkeldey, Stockmair & Schwanhäusser Anwaltssozietät

(57) **Zusammenfassung**

Die Erfindung betrifft eine rohrförmige, wachstumsfähige Stützprothese, aufweisend ein Maschengerüst, wobei das Maschengerüst zumindest zwei miteinander über Verbindungsglieder verbundene, punktsymmetrisch um die Stützprothesenlängsachse angeordnete Gerüstringe aufweist, wobei die Gerüstringe und/oder die Verbindungsglieder zumindest eine Sollbruchstelle aufweisen.

## Beschreibung

Die vorliegende Erfindung betrifft wachstumsfähige Stützprothesen. Insbesondere betrifft die vorliegende Erfindung Stützprothesen zum Abstützen eines Gefäßes von innen als auch herzklappentragende oder blutgefäßtragende Stützprothesen, welche "tissue engineertes" autologes oder heterologes Gewebe oder körperfremdes, aber dezellularisiertes oder fixiertes Herzklappengewebe umfassen können. Solche Stützprothesen können auch "Stents" genannt werden.

Stents finden breite Anwendung zum Abstützen von verengten oder beschädigten Gefäßen zum Beispiel nach kardiologischen interventionellen Eingriffen oder nach invasiven chirurgischen Eingriffen. Auch bei kongenitalen Herzfehlern finden Stents seit ihrer Einführung Ende der siebziger Jahre sehr breite Anwendung.

Bei der Behandlung von Kindern, beispielsweise neugeborenen Kindern mit kongenitalen Herzerkrankungen, werden Stents in einer der Größe des Gefäßes des Kindes angepassten Größe eingesetzt. So muss ein im Kindesalter implantierter Stent infolge des späteren Wachstums des Kindes entweder mittels Ballon-Angioplastie wiederholt aufgedehnt werden oder mit einem oder mehreren chirurgischen Eingriffen durch größere Stents ersetzt werden, um eine Verengung zu verhindern.

Diesem Problem ist im Stand der Technik auf unterschiedliche Art und Weise begegnet worden. Es gibt Stents, die aus im Körper abbaubaren Materialien bestehen. Solche biologisch abbaubaren, oder bioabsorbierbaren Stents können kurzzeitig nach dem Implantieren in ein Gefäß ihre Stützfunktion entfalten und verschwinden danach allmählich. Bekannte abbaubare Materialien für diesen Zweck sind Polymere oder Metalle. Solche bioabbaubaren Stents sind beispielsweise in Erne et al. (2006), Cardiovasc. Intervent. Radiol. 29, 11-16, beschrieben.

Biologisch abbaubare Stents bringen jedoch einige Nachteile mit sich. Zum einen geht mit dem Verschwinden der Stentstruktur naturgemäß auch ein Verlust der damit verbundenen Stützfunktion einher. Dieser Verlust stellt insbesondere bei kongenitalen Gefäßanomalien ein besonders schwieriges Problem dar, da die gewünschte Stützfunktion zum einen nicht ausreichend und/oder nur kurzzeitig vorhanden ist, obwohl sie vorwiegend langfristig benötigt wird. Zum anderen werden vor allem bei dem Auflösen von Stents aus biologisch abbaubaren Polymeren zum Teil schwerwiegende inflammatorische und/oder Fremdkörper-Reaktionen an der Gefäßwand beobachtet.

Aufgabe der vorliegenden Erfindung ist es daher, eine Stützprothese bereitzustellen, die die gewünschte Stützfunktion auch im wachsenden Organismus über längere Zeiträume entfalten kann, wobei die oben erwähnten Nachteile weitgehendst vermieden werden sollen.

Erfindungsgemäß wird diese Aufgabe durch eine rohrförmige, unter Umständen herzklappentragende, wachstumsfähige Stützprothese gelöst, die ein Maschengerüst aufweist, wobei das Maschengerüst zumindest zwei miteinander über Verbindungsglieder verbundene, punktsymmetrisch um die Stützprothesenlängsachse angeordnete Gerüstringe aufweist, wobei die Gerüstringe und/oder die Verbindungsglieder zumindest eine Sollbruchstelle aufweisen.

Die Erfinder haben überraschenderweise gefunden, dass das Vorsehen zumindest einer Sollbruchstelle im Gerüstring und/oder im Verbindungsglied der Stützprothese ein langfristiges Wachstum dieser Stützprothese ermöglicht, ohne dass die gewünschte Stützfunktion erheblich reduziert wird. Wird das zu stützende Gefäß oder der zu stützende Herzklappenbereich beispielsweise eines Kleinkinds mit der Zeit größer, so kann die erfindungsgemäße Stützprothese mitwachsen, da die Sollbruchstellen aktiv oder passiv gesprengt werden können. Für den Fall, dass die erfindungsgemäße wachstumsfähige Stützprothese zusätzlich eine Herzklappe, zum Beispiel eine tissue-engineerte ("TE") Herzklappe aufweist, hat dies den zusätzlichen Vorteil, dass die Herzklappe in analoger Weise wie auch die Stützprothese mitwachsen kann. Die erfindungsgemäße Stützprothese kann vor dem Implantieren in einen Patienten mit homologen, vorzugsweise mit autologen Zellen, vorzugsweise Endothelzellen, Fibroblasten und/oder Myofibroblasten und/oder weitere pluripotente Progenitorzellen und/oder Stammzellen besiedelt werden oder mit bereits *in vitro* hergestelltem Gewebe ergänzt werden. Diese Zellen bilden nach wenigen Wochen mit der Gefäßwand des zu stützenden Gefäßes ein einheitliches Gewebe mit "integrierter" Stützprothese. Mit zunehmendem Lumen des Gefäßes wirken Kräfte auf die Stützprothese, die an den Sollbruchstellen des Stützprothese-Maschengerüsts zu punktuellen Brüchen führen können, d. h. das Maschengerüst der Stützprothese kann durch die wachstumsbedingten, radial wirkenden Kräfte an seinen Sollbruchstellen auseinander gesprengt werden. So kann sich die erfindungsgemäße Stützprothese (und ggf. auch eine darin enthaltene Herzklappe) automatisch an einen größer gewordenen Gefäßlumenumfang anpassen, wobei eine hinreichende Stützfunktion durch das Zusammenspiel des die Stützprothese besiedelnden Zellengewebes sowie mit den noch vorhandenen Fragmenten des Maschengerüstes gewährleistet wird.

Der Begriff "Sollbruchstelle" ist generell so zu verstehen, dass an dieser Stelle im Gerüstring und/oder im Verbindungsglied nicht nur ein Bruch bzw. eine Sprengung bzw. ein Auseinanderbrechen der Sollbruchstelle sondern auch eine Dehnung der Sollbruchstelle möglich ist. Eine Dehnung der Sollbruchstelle ist als eine plastische Deformation dieser aufzufassen, bei der sich die Geometrie der Sollbruchstelle ändert, ohne dass es zu einer Trennung der der Sollbruchstelle benachbarten Teile der Stützprothese kommen muss. Die Dehnung einer Sollbruchstelle kann auch als eine Vorstufe des finalen Bruchs der Sollbruchstelle auftreten. Insoweit schließen sich die Interpretationen einer Sollbruchstelle einerseits als Bruchpunkt, anderseits als Dehnungspunkt nicht gegenseitig aus. Obwohl im nachfolgenden Text überwiegend von Sollbruchstellen im Sinne eines Bruchpunktes gesprochen wird, ist es dem Fachmann somit klar, dass unter Umständen darunter (auch) ein Dehnungspunkt zu verstehen ist.

Es ist möglich, die Sollbruchstellen so auszugestalten, dass sich diese mit der Zeit auflösen, um an den so entstandenen Schwachstellen eine Sprengung, oder ein Auseinanderbrechen, infolge des Wachstumsprozesses zu ermöglichen (passive Sprengung). Auch vorstellbar ist eine aktive Aufsprengung der Sollbruchstellen, beispielsweise mittels Ballon-Angioplastie, um eine bereits implantierte Stützprothese an ein seit dem Implantieren größer gewordenes Lumen anzupassen.

Die Sollbruchstellen können auch so ausgestaltet werden, dass sie unter äußerer Einwirkung, beispielsweise unter Einwirkung von Schallwellen wie z.B. Ultraschall oder Stoßwellen, Röntgenstrahlen, magnetischer Energie, usw. gesprengt oder zumindest geschwächt werden können. Eine derartige Ausgestaltung der Sollbruchstellen hat den besonderen Vorteil, dass die Sprengung einer Stützprothese, die beispielsweise bereits im frühen Kindesalter implantiert worden ist, später von außen herbeigeführt oder zumindest begünstigt werden kann, ohne dass das mittlerweile gewachsene Kind nochmals operiert werden muß.

Unabhängig davon, ob die Stützprothese aktiv oder passiv gesprengt wird, ermöglichen die Sollbruchstellen der erfindungsgemäßen Stützprothese, dass die Größe, z. B. der Durchmesser der erfindungsgemäßen Stützprothese, an die Größe des Gefäßes angepasst wird.

Gemäß einer Ausführungsform der vorliegenden Erfindung weist zumindest ein Gerüstring des Maschengerüsts n periodische, längs der Stützprothesenlängsachse sich erstreckende, Hoch- und Tiefpunkte bildende Verformungen auf, die eine sich auf die Stützprothesenlängsachse bezogene Amplitude A aufweisen, wobei n = 16-70, bevorzugt 20-56, besonders bevorzugt 24-42. Hierbei ist A als die maximale Auslenkung einer jeweiligen Verformung aus ihrer Mittellage, also aus einer für jeden Gerüstring definierten Umfangsmittellinie M (wobei die durch M gebildete Fläche die Stützprothesenlängsachse senkrecht schneidet) zu verstehen, und beträgt 0,25-8 mm, bevorzugt 0,75-4 mm, besonders bevorzugt 1-2 mm. Diese Verformungen, die sinusförmig, rechteckig, sägezahnförmig, dreieckig oder mäanderförmig, vorzugsweise sinusförmig, ausgestaltet sein können, verleihen der Stützprothese ihre für das Einführen mittels Katheter notwendige Faltbarkeit und Elastizität, d. h. sie ermöglichen, dass der Durchmesser der Stützprothese vor dem Einführen in einen Patienten auf den benötigten Durchmesser reduziert werden kann, ohne die Stützprothese in ihrer Gesamtlänge wesentlich zu verkürzen.

In einer bevorzugten Ausführungsform weist das Maschengerüst 2-8, vorzugsweise 2-6, besonders bevorzugt 2-4 Gerüstringe auf.

Gemäß einer weiteren Ausführungsform der vorliegenden Erfindung ist die Anzahl der periodischen Verformungen zweier jeweils benachbarter Gerüstringe identisch oder die Anzahl der Verformungen zweier jeweils benachbarter Gerüstringe unterscheidet sich um ein ganzzähliges Vielfaches voneinander. Ist die Anzahl der Verformungen zweier jeweils benachbarter Gerüstringe identisch, so kann jede Verformung eines Gerüstringes mit jeder oder beispielsweise mit jeder zweiten, dritten oder vierten korrespondierenden Verformung eines jeweils benachbarten Gerüstringes über die Verbindungsglieder verbunden werden, um das Maschengerüst zu bilden. Dies hat den Vorteil, dass eine Stützprothese mit einer sehr regelmäßigen Maschenstruktur entsteht, die die Innenwand eines Gefäßes an jedem Punkt über die Gesamtlänge der Stützprothese hinweg mit gleicher Kraft abstützt. Unterscheidet sich die Anzahl der Verformungen zweier jeweils benachbarten Gerüstringe um ein ganzzähliges Vielfaches voneinander, so kann jede Verformung eines Gerüstringes mit jeder zweiten, jeder dritten, jeder vierten usw. Verformung eines jeweils benachbarten Gerüstringes über Verbindungsglieder verbunden werden. In dieser Weise kann die Dichte des Maschengerüstes über die gesamte Länge der Stützprothese hinweg unterschiedlich ausgestaltet werden, um unterschiedlichen klinischen Anforderungen zu genügen, bei denen entlang eines gestützten Abschnittes eines Gefäßes unterschiedliche Stützkräfte erforderlich sind.

So kann die Stützprothese gemäß dieser Ausführungsform der Erfindung so ausgestaltet sein, dass durch geeignete Auswahl der Anzahl der Verformungen jeweils benachbarter Gerüstringe die radiale Stützkraft der Stützprothese von einem Ende zum anderen Ende kontinuierlich ab- oder zunimmt. Auch denkbar gemäß dieser Ausführungsform der Erfindung sind Ausgestaltungen, bei denen die radiale Stützkraft einer Prothese entlang der Stützprothesenlängsachse zur Mitte der Stützprothese abnimmt, d. h. bei denen diese Stützkraft an beiden Enden der Stützprothese jeweils größer ist als in der Mitte. Umgekehrt sind andere Ausgestaltungen möglich, bei denen die Dichte des Maschengerüsts durch geeignete Auswahl der Perioden der Verformungen jeweils benachbarter Gerüstringe derart gewählt wird, dass die Stützkraft entlang der Stützprothesenlängsachse in der Mitte der Stützprothese am größten ist.

Gemäß einer weiteren Ausführungsform der vorliegenden Erfindung können zwei benachbarte Gerüstringe derart phasenversetzt zueinander angeordnet sein, dass Hochpunkte eines Gerüstringes mit Tiefpunkten eines jeweils benachbarten Gerüstringes über die Verbindungsglieder verbunden sind. Bei vielen im Stand der Technik bekannten Gefäßstützprothesen sind zwei benachbarte, periodisch verformte Ringe der Prothesenstruktur über ihre jeweiligen Tief- oder Hochpunkte verbunden, also wird der Hochpunkt eines Gerüstringes mit dem Hochpunkt eines jeweils benachbarten Gerüstringes verbunden oder der Tiefpunkt eines Ringes wird mit dem Tiefpunkt eines jeweils benachbarten Ringes verbunden. Werden jedoch zwei benachbarte Gerüstringe punktsymmetrisch um die Stützprothesenlängsachse gegeneinander so rotiert, so dass der Hochpunkt eines verformten Gerüstringes dem Tiefpunkt eines jeweils benachbarten verformten Gerüstringes gegenüberliegt, so kann der Hochpunkt des einen Ringes mit dem Tiefpunkt des jeweils benachbarten Ringes über ein Verbindungsglied verbunden werden, wodurch eine sehr hohe Flexibilität des gesamten Maschengerüstes und eine damit verbundene erhöhte Wachstumsfähigkeit der Stützprothese erzielt werden können. Gemäß einer weiteren Ausführungsform ist es möglich, einen Hochpunkt eines Gerüstringes mit einem Mittelpunkt, d. h. mit einem Punkt an oder neben der Mittellinie M wie oben definiert, des jeweils benachbarten Gerüstringes zu verbinden. Gemäß einer weiteren Ausführungsform ist es möglich, einen Mittelpunkt, d. h. einen Punkt an oder neben der Mittellinie M, eines Gerüstringes mit einem Mittelpunkt des jeweils benachbarten Gerüstringes zu verbinden. Gemäß einer weiteren Ausführungsform ist es möglich, einen Tiefpunkt eines Gerüstringes mit einem Mittelpunkt, d. h. mit einem Punkt an oder neben der Mittellinie M, des jeweils benachbarten Gerüstringes zu verbinden.

Gemäß einer weiteren Ausführungsform der vorliegenden Erfindung können die Verbindungsglieder als Ringe, Klemmen oder sich parallel zur Hauptachse der Stützprothese (auch "Stützprothesenlängsachse" genannt) erstreckende Schlaufen, Fäden, Drähte oder Streben ausgestaltet sein.

Dabei können die Verbindungsglieder mit den jeweils benachbarten Gerüstringen locker oder starr verbunden sein. Durch ein lockeres Anbringen, beispielsweise durch Polymer oder Metalle, die weicher als das Grundmaterial der Stützprothese sind, kann eine sehr hohe Flexibilität des gesamten Maschengerüstes erzielt werden. Eine solche lockere Verbindung der Gerüstringe kann auch durch Draht, einen Clip oder Nahtmaterial bewerkstelligt werden. Es können auch an Verbindungsgliedern Sollbruchstellen vorgesehen sein.

Dagegen wirkt sich ein starres Verbinden der Verbindungsglieder mit ihren jeweiligen Gerüstringen aussteifend auf das Maschengerüst aus. Bevorzugt sind die Verbindungsglieder, die zwei jeweils benachbarte Gerüstringe verbinden, als starre Streben ausgestaltet, die vorzugsweise in die Struktur der jeweils benachbarten Gerüstringe nahtlos integriert sind. In dem Fall, dass die wachstumsfähige Stützprothese aus einem Röhrenrohling beispielsweise mittels eines Lasers herausgeschnitten werden soll, können solche starren Streben oder Stege auf einfache und dem Fachmann bekannte Weise dadurch geschaffen werden, dass der Rohling an den Stellen, wo später Verbindungsglieder vorgesehen sind, nicht geschnitten wird.

Es ist aber auch denkbar, dass in einem Abschnitt der Stützprothese lockere Verbindungsglieder, dahingegen in einem anderen Abschnitt der Stützprothese starre Verbindungsglieder eingesetzt werden. Auch ist eine Mischung von lockeren und festen Verbindungsgliedern über die gesamte Struktur des Maschengerüsts hinweg denkbar.

Gemäß einer weiteren Ausführungsform der vorliegenden Erfindung bestehen die Gerüstringe und/oder die Verbindungsglieder zumindest zum Teil aus einem biologisch abbaubaren Material, beispielsweise aus einem biologisch abbaubaren Metall wie beispielsweise einem Edelstahl oder einem biologisch abbaubaren Polymer.

Bestehen die Gerüstringe und/oder die Verbindungsglieder nur zum Teil aus einem biologisch abbaubaren Material, so löst sich dieses Material im Laufe der Zeit nach dem Implantieren *in vivo* auf, so dass lediglich die Teile der Gerüstringe und/oder Verbindungsglieder, die aus nicht abbaubarem Material bestehen, fortbestehen. So wird die gewünschte unterstützende Funktion der Stützprothese langfristig von den zurückgebliebenen, nicht abbaubaren Teilen der Gerüstringe und/oder Verbindungsglieder im Zusammenspiel mit dem zwischenzeitlich entstandenen Zellgewebe übernommen. Bestehen die Gerüstringe und/oder Verbindungsglieder ganz aus biologisch abbaubarem Material, so verschwindet das gesamte Material des Maschengerüstes mit der Zeit, so dass nur noch das zwischenzeitlich entstandene Zellgewebe der Zellen, die die Stützprothese anfangs besiedelt haben, die gewünschte Stützfunktion übernimmt. Für manche Patienten kann eine solche Stützfunktion durchaus ausreichen. Die Auswahl der Materialien, d. h. ob die erfindungsgemäße Stützprothese ganz, zum Teil oder überhaupt nicht aus biologisch abbaubarem Material besteht, richtet sich in der Regel nach dem zu behandelnden Patienten. Generell kann aber gesagt werden, dass bei erhöhtem Bedarf an Stützfunktion Ausgestaltungen mit höheren Anteilen an nicht biologisch abbaubaren Materialien eher vorzuziehen sind, denn solche erfindungsgemäßen Stützprothesen sind imstande, auch nach dem Wachstum, d.h. nachdem das Maschengerüst an seinen Sollbruchstellen zumindest teilweise auseinandergesprengt worden ist, eine hohe radiale Stützkraft auszuüben.

Gemäß einer weiteren Ausführungsform der Erfindung besteht zumindest ein Teil der Gerüstringe sowie der Verbindungsglieder der Stützprothese aus einem Polymer. Hier kann die gesamte Stützprothese aus demselben Polymer bestehen, oder es können die Gerüstringe sowie die Verbindungsglieder aus einem Polymer und die Sollbruchstellen aus einem anderen Polymer bestehen. So können beispielsweise die Gerüstringe und die Verbindungsglieder aus einem nicht oder nur schwer biologisch abbaubaren Polymer, dahingegen die Sollbruchstellen aus einem oder mehreren biologisch abbaubaren Polymeren bestehen. Durch eine solche Ausgestaltung können die Wachstumseigenschaften, wie unten näher erläutert, gesteuert werden.

Nach einer weiteren Ausführungsform der Erfindung weist eine solche Stützprothese aus Polymeren an ihrer Innenseite eine Herzklappe, beispielsweise eine mittels "Tissue Engineering" mit autologem Gewebe hergestellte Herzklappe oder ein tissue engineertes Gefäß auf. Neben dem stark verminderten Risiko einer Immunabstoßung sind solche mittels "Tissue Engineering" hergestellten Herzklappen auch zur Anwendung in einer wachstumsfähigen Stützprothese hervorragend geeignet, da sie naturgemäß mitwachsen können.

Nach einer weiteren Ausführungsform kann die wachstumsfähige Stützprothese eine herzklappentragende Stützprothese sein. Hier kann die Herzklappe und/oder die Stützprothese selbst tissue engineertes, autologes oder heterologes Gewebe, oder körperfremdes Gewebe, dezellularisiertes oder fixiertes Herzklappengewebe umfassen. Im Idealfall umfasst sowohl die erfindungsgemäße Stützprothese sowie die ggf. vorhandene Herzklappe dieselbe Gewebeart, die vorzugsweise autologes Gewebe ist. Eine solche Herzklappe kann mit der Stützprothese mitwachsen.

Als geeignete nicht oder nur schwer biologisch abbaubare Polymere kommen vor allemPTFE, Dacron, PHA und Poly-3-hydroxybutyrat P3HB in Betracht.

Unabhängig davon, aus welchem Material die Stützprothese besteht, kann die Stützprothese wie oben beschrieben bevorzugt mit körpereigenen Zellen besiedelt werden, die so besiedelte Stützprothese wird auf die für die Implantation benötigte Größe bzw. auf den benötigten Durchmesser zusammengefaltet ("gecrimpt", vom englischen "crimp", knautschen) und die so zusammengedrückte Stützprothese wird beispielsweise über einen Gefässkatheter an den gewünschten Ort im Körper gebracht. So kann die Stützprothese ihre Stützfunktion im Gefäß nach gegebenenfalls erforderlichem Expandieren mittels eines Ballonkatheters ausüben. Dies gilt sowohl für Stützprothesen mit als auch ohne Herzklappen.

Gemäß einer weiteren Ausführungsform der vorliegenden Erfindung wird das biologisch abbaubare Material aus zumindest einer Legierung, zumindest einem Edelstahl und/oder zumindest einem Polymer mit Formgedächtnis ausgewählt. Als Legierungen mit Formgedächtnis kommen vor allem beispielsweise Nickel-Titan-Legierungen, wie beispielsweise das bekannte Nitinol, oder Aluminiumlegierungen, Magnesiumlegierungen oder Eisenlegierungen in Betracht. Als Polymere mit Formgedächtnis kommen beispielsweise tert-Butylacrylat oder Poly(ethylenglycol)dimethylacrylat oder PCL kombiniert mit 2,4-Toluoldüsocyanat ethylenglycol in Betracht.

Als biologisch abbaubare Polymere kommen vor allem Polyglykolsäure (PGA), Polymilchsäure (PLA), Polyhydroxyalkanoat (PHA) und Poly-4-hydroxybutyrat (P4HB), Polycaprolactone (PLGA), Polycarbonate, Polyamide, Polyanhydride, Polyaminosäuren, Polyorthoester, Polyacetate, Polycyanoacrylate sowie abbaubare Polyurethane und nicht erodierbare Polymere wie Polyacrylate, Ethylenvinylacetat-Polymere und andere substituierte Zelluloseacetate sowie Derivate davon in Betracht. Polyester werden hierbei bevorzugt. Bevorzugte biologisch abbaubare Polymere umfassen Polymere folgender Gruppen: Polyester der Hydroxycarboxysäuren, Polyanhydride der Dicarboxyester, und Copolymere der Hydroxycarboxysäuren und der Dicarboxyester.

In einer weiteren Ausführungsform besteht das Material aus einem synthetischen Polymer aus mindestens einem der folgenden Monomere: Glykolid, Laktid, p-Dioxanon, Caprolacton, Trimethylencarbonat, Butyrolacton. In besonderen Ausführungsformen wird das Material ausgewählt aus einer Gruppe bestehend aus Polymeren oder Copolymeren von Glycolsäure, Milchsäure und Sebacinsäure. Polyglykolsäurepolymere werden hierbei bevorzugt.

Diese Polymere können sowohl rein als auch in Mischungen aus zwei oder mehreren der genannten Substanzen oder Mischungen dieser Substanzen mit weiteren biologisch abbaubaren Polymeren verwendet werden. In einer bevorzugten Ausführungsform wird ein Mischpolymer aus 80-98% PGA und 20-2% PHA verwendet.

In einer weiteren besonderen Ausführungsform umfasst das biologisch abbaubare Material ein Polyhydroxyalkanoat (PHA). PHA kann dabei mit einem weiteren nicht abbaubaren Polymer beschichtet werden oder selbst als Beschichtung dienen. Ein bevorzugtes Polyhydroxyalkanoat für diese Verwendung wird *in vivo* innerhalb von weniger als 9 Monaten, noch bevorzugter in weniger als 6 Monaten und am stärksten bevorzugt in weniger als 3 Monaten abgebaut. Eine bevorzugte Zusammensetzung der Polyhydroxyalkanoate beinhaltet 2-, 3-, 4- oder 5-Hydroxysäuren, z. B. Poly-4-hydroxybutyrate. Weiterhin kann die Zusammensetzung ein Poly-4-hydroxybutyrat-co-3-hydroxybutyrat sowie Kombinationen davon beinhalten. Am stärksten bevorzugt wird dabei Poly-4-hydroxybutyrat.

In einer weiteren besonderen Ausführungsform umfasst das biologisch abbaubare Material Homopolymere und Copolymere mit einer beliebigen Kombination folgender Monomere: 3-Hydroxybutyrate, 3-Hydroxyvalerat, 3-Hydroxypropionat, 2-Hydroxybutyrat, 4-Hydroxybutyrat, 4-Hydroxyvalerat, 3-Hydroxyhexanoat, 3-Hydroxyheptanoat, 3-Hydroxyoctanoat, 3-Hydroxynonanoat, 3-Hydroxytridecanoat, 3-Hydroxytetradecanoat, 3-Hydroxypentadecanoat, 3-Hydroxyhexadecanoat, 3-Hydroxyheptadecanoat und 3-Hydroxyoctadecanoat.

Nach einer weiteren Ausführungsform der Erfindung kann die Stützprothese aus einem nicht biologisch abbaubarem Material beschaffen sein und mit einem oder mehreren biologisch abbaubaren Materialien, z.B. Polymeren beschichtet werden. Wiederum ist es nach einer weiteren Ausführungsform der Erfindung auch möglich, dass die Stützprothese aus einem biologisch abbaubaren Material beschaffen, und mit einem oder mehreren nicht biologisch abbaubaren Materialien beschichtet ist. Sowohl das biologisch abbaubare Material als auch das nicht biologisch abbaubare Material können ein Polymer oder ein Metall sein.

Gemäß einer weiteren Ausführungsform der vorliegenden Erfindung ist die zumindest eine Sollbruchstelle bevorzugt an oder neben dem Mittelpunkt zwischen einem Hoch- und Tiefpunkt des periodisch verformten Gerüstringes, d. h. an oder neben dem Schnittpunkt des Gerüstringes mit der Mittellinie M wie oben definiert angeordnet. Die zumindest eine Sollbruchstelle kann aber auch zusätzlich oder alternativ dazu an den Hoch- und/oder Tiefpunkten der periodischen Verformungen des Gerüstringes angeordnet sein.

Beim Wachsen der Stützprothese gemäß der Erfindung werden radial von innen nach außen strebende Kräfte auf das Maschengerüst der Stützprothese ausgeübt. Wird die Stützprothese während des Wachstumsvorgangs ausgedehnt, so konzentrieren sich diese Kräfte an den Hoch- und Tiefpunkten der Gerüstringe, da diese Hoch- und Tiefpunkte der Stützprothese insgesamt einen großen Teil ihres Flexionsvermögens verleihen. Werden die Sollbruchstellen ausschließlich an den Hoch- und Tiefpunkten des periodisch verformbaren Gerüstringes angeordnet, besteht die Gefahr, dass das gesamte Maschengerüst frühzeitig gesprengt wird, wodurch unter Umständen einen Großteil seiner Stützfunktion in unerwünschter Weise verlorenginge. Werden die Sollbruchstellen aber ausschließlich oder vorwiegend an oder neben dem Mittelpunkt zwischen den Hoch- und Tiefpunkten des periodisch verformbaren Gerüstringes angeordnet, kann unter Umständen das Auseinanderbrechen des Maschengerüsts verzögert werden.

Durch das gezielte Mischen der Orte, an denen Sollbruchstellen angeordnet sind, kann man die Sprengneigung der Stützprothese in sehr vorteilhafter Weise steuern. Je mehr Sollbruchstellen an Hoch- und/oder Tiefpunkten eines periodisch verformbaren Gerüstringes angeordnet sind, desto größer ist die Sprengneigung. Je mehr Sollbruchstellen an oder neben den Mittelpunkten eines periodisch verformbaren Gerüstringes angeordnet sind, desto geringer ist die Sprengneigung.

Die konkrete Ausgestaltung der Sollbruchstellen ist nicht beschränkt, so lange sie ihre Funktion wie oben beschrieben ausüben können. Konkret lässt sich diese Funktion auf unterschiedliche Art und Weise realisieren. Bei einer Stützprothese aus einem metallischen Material kann eine Sollbruchstelle beispielsweise als zumindest eine Perforation in einer Gerüstringsstrebe oder im Verbindungsglied ausgestaltet sein. Solche Perforationen können unterschiedlicher Art sein, beispielsweise als eines oder mehrere durchgehende Löcher und/oder als eine oder mehrere nicht durchgehende Vertiefungen im Stützprothesenmaterial. Die Form der Löcher bzw. der Vertiefungen ist nicht eingeschränkt: sie kann rund, elliptisch, polygonal, rechteckig und/oder quadratisch sein. Die Vertiefung kann auch als eine Kerbe oder mehrere Kerben im Material der Stützprothese ausgestaltet sein, die zum Beispiel quer oder schräg über die Breite einer Strebe eines Gerüstringes und/oder eines Verbindungsgliedes verläuft. Es ist auch möglich, solche Kerben, oder Verengungen, auf beiden Seiten einer Strebe eines Gerüstringes und/oder Verbindungsgliedes so anzuordnen, dass zwei Kerben dieselbe Sollbruchstelle von beiden Seiten des Gerüstrings bzw. des Verbindungsglieds (d. h. von oben und unten) definieren, d. h. sowohl von den der Gefäßwand als auch der Wand des Gefäßlumens zugewandten Seiten der Stützprothese. Alternativ oder auch ergänzend dazu können an der selben Stelle, d. h. in der selben Höhe der Strebe wie die zwei oben und unten angeordneten Kerben, eine oder zwei Kerben rechts und links angeordnet sein. So kann die Stärke einer Sollbruchstelle dahingehend eingestellt werden, dass man sie aus einer, zwei, drei oder vier Kerben, die alle in derselben Höhe der Strebe oder des Verbindungsglieds angeordnet sind, schafft. Obwohl diese Ausführungsform eine Strebe und/oder ein Verbindungsglied mit vierkantigem Querschnitt annimmt, ist anzumerken, dass Entsprechendes bei Streben und/oder Verbindungsgliedern mit anders ausgestalteten Querschnitten gilt.

Es ist auch möglich eine Sollbruchstelle so auszugestalten, dass sie bei einer punktuellen oder linienförmigen Überschreitung der plastischen Verformbarkeit einer Strebe des Gerüstringes auseinanderbricht, und/oder zur Schaffung einer Sollbruchstelle punktuelle oder linienförmige Unterschiede in der Strukturdichte des Stützprothesenmaterials vorzusehen. Sollbruchstellen können auch durch das Schaffen punktueller oder linienförmiger Schädigungen in der Oberflächenstruktur oder der inneren Struktur des Gerüstringes und/oder des Verbindungsgliedes (beispielsweise durch lokale Erhöhung der Porosität des Materials, beispielsweise durch die Aufnahme von Nanopartikeln im Bereich der Sollbruchstelle) bewerkstelligt werden. Auch vorstellbar ist eine örtlich begrenzte oder unbegrenzte Mischung des Grundmaterials der Stützprothese mit Polymer und/oder mit Polymerinterponaten, Drähten, Fäden und/oder organischen Interponaten wie beispielsweise Kollagen.

Eine Sollbruchstelle kann auch als Schnitt oder Spalt im Material der Stützprothese (d h. im Gerüstring oder im Verbindungsglied) ausgestaltet sein. Dieser Schnitt oder Spalt kann mit einem nicht metallischen Material, beispielsweise einem biologisch abbaubaren Material im Bereich der Sollbruchstelle überdeckt sein, so dass zunächst nur das biologisch abbaubare Material die Sollbruchstelle zusammenhält. Dies kann beispielsweise so erreicht werden, dass das biologisch abbaubare Material beidseitig des Schnitts oder Spalts auf dem Material des Gerüstringes oder des Verbindungsgliedes überlappt und mit diesem verbunden ist. Löst sich das biologisch abbaubare Material mit der Zeit im Körper auf, so reduziert sich seine Stützfunktion entsprechend, bis entweder die infolge des Wachstums entstehenden Sprengkräfte das Stützvermögen des biologisch abbaubaren Materials übersteigen oder sich das biologisch abbaubare Material ganz auflöst und den darunterliegenden Schnitt oder Spalt freilegt, so dass die Stützprothese ungehindert wachsen (oder mittels Ballon-Angioplastie aufgedehnt werden) kann. Durch geeignete Auswahl des an der Sollbruchstelle verwendeten, biologisch abbaubaren Materials kann die Geschwindigkeit des Wachstumsprozesses gesteuert werden, denn es ist bekannt, dass unterschiedliche biologisch abbaubare Materialien sich unterschiedlich schnell *in vivo* auflösen.

Wie oben erwähnt, kann die zumindest eine Sollbruchstelle auch so ausgestaltet sein, dass sie unter äußerer Einwirkung gesprengt, aufgelöst, geschwächt oder gedehnt werden kann. Die äußere Einwirkung kann aus Schallwellen, zumindest einem magnetischen Feld, einer Kombination aus magnetischem Feld und elektromagnetischem Feld; elektromagnetischer Strahlung, elektrischer Energie und einer beliebigen Kombination davon ausgewählt sein. Die äußere Einwirkung kann von innerhalb oder außerhalb des Körpers erfolgen, sie erfolgt zumindest von außerhalb der Stützprothese. Erfolgt die äußere Einwirkung von innerhalb des Körpers, so kann sie beispielsweise mit Hilfe eines intravaskulären Katheters oder minimal invasiv mit Hilfe der Endoskopie erfolgen. Sie Schallwellen können beispielsweise Ultraschall und/oder Stoßwellen sein. Die Kombination aus magnetischem Feld und elektromagnetischem Feld kann beispielsweise Kernspinresonanz (Magnetic Resonance Imaging, MRI) sein. Die elektromagnetische Strahlung kann beispielsweise Röntgenstrahlung oder Infrarotstrahlung (thermische Energie) sein. Zum Beispiel kann durch eine lokale Temperaturerhöhung ein steiler Abfall der Steifheit einer Sollbruchstelle (Dehnung der Sollbruchstelle durch plastische Deformation) oder sogar ein Bruch der Sollbruchstelle erreicht werden.

Nach einer weiteren Ausführungsform der Erfindung kann die Sollbruchstelle so ausgestaltet sein, dass ihre Rigidität oder Haltvermögen erst ab einer Beanspruchung von vorbestimmter Dauer bzw. von vorbestimmter Stärke nachlässt. Beispielsweise kann die Sollbruchstelle einer für den Aortenbereich gedachten wachstumsfähigen Stützprothese so beschaffen sein, dass der Schwellenwert der maximal tolerierten, durch das Pumpen des Herzens verursachten mechanischen Beanspruchung des Sollbruchstellenmaterials erst ab einer bestimmter Anzahl von Herzschlägen erreicht wird. Ausgehend von einer durchschnittlichen Herzschlagrate kann man so effektiv die Dauer vorprogrammieren, nach der die Stützprothese nach der Implantation anfängt, sich aufzulösen oder sich zu dehnen. Dies ermöglicht eine Steuerung der Stützprothesenwachstumsrate, was insbesondere bei dem Einsatz im Kleinkind vor Vorteil ist, da dadurch die Anzahl an chirurgischen Eingriffen wesentlich reduziert wird. Es ist natürlich auch vorstellbar, dass die Beanspruchung, der die Sollbruchstelle ausgesetzt werden soll, einer anderen Natur als mechanisch ist.

Da die Menge des biologisch abbaubaren Materials an den Sollbruchstellen in der Regel sehr gering ist, sind bei dessen Auflösung keine Entzündungsreaktionen zu befürchten, wie im Stand der Technik bei dem Auflösen von Stützprothesen, die ganz aus biologisch abbaubarem Material bestehen, der Fall ist.

Eine entsprechende Ausgestaltung unter Verwendung eines biologisch abbaubaren Materials ist auch dann möglich, wenn statt eines Schnitts oder eines Spalts, eine Perforation und/oder eine Kerbe im Material der Stützprothese vorliegen.

Es ist auch möglich, unabhängig von der Ausgestaltung als Perforation, Kerbe, Vertiefung, Spalt oder Schnitt an der zumindest einen Sollbruchstelle mehrere Schichten unterschiedlicher biologisch abbaubarer Materialien anzubringen, so dass eine zeitlich gestaffelte Aufsprengung der Stützprothese ermöglicht wird. So kann beispielsweise gewährleistet werden, dass die Auflösung der Sollbruchstelle anfangs sehr langsam verläuft und erst nach langer Zeit (d. h. nachdem die langsam sich auflösende äußere Schicht verschwunden ist) die Auflösungsrate der Sollbruchstelle und das damit verbundene Wachstumspotential der Stützprothese zunimmt, oder umgekehrt.

Es ist auch möglich, dass eine Stützprothese eine Kombination unterschiedlich ausgestalteter Sollbruchstellen aufweist. Zum Beispiel kann eine Stützprothese eine oder mehrere Sollbruchstellen als Perforationen ohne biologisch abbaubares Material und eine oder mehrere Sollbruchstellen als mit einem oder mehreren biologisch abbaubaren Materialien überdeckte Schnitte und/oder Spalte aufweisen. So kann gewährleistet werden, dass unterschiedliche Bereiche derselben Stützprothese innerhalb derselben Zeit *in vivo* unterschiedlich schnell wachsen. Die erfindungsgemäße Stützprothese erlaubt daher einen sehr hohen Grad an Flexibilität bei der Anpassung an den unterschiedlichsten physiologischen Anforderungen.

Es ist auch möglich, im Bereich der Sollbruchstellen nicht biologisch abbaubares Polymer zu verwenden. Die Sollbruchstelle kann wie oben beschrieben ausgestaltet sein, d. h. als Perforation, Kerbe, Vertiefung, Spalt oder Schnitt. Wird beispielsweise beidseitig überlappend über einen Schnitt oder einen Spalt im Gerüstring und/oder im Verbindungsglied nicht biologisch abbaubares Polymer gebracht, so hängt die Stärke der Sollbruchstelle und damit auch das Wachstumsvermögen der Stützprothese von der Stärke des nicht biologisch abbaubaren Polymers ab. Bei bekannter Materialstärke des nicht biologisch abbaubaren Polymers kann in dieser Weise die Stärke bzw. die Wachstumseigenschaften der Stützprothese je nach physiologischen Anforderungen gesteuert werden.

Die Sollbruchstellen weisen also nach einer bevorzugten Ausführungsform ein nicht metallisches Material, beispielsweise ein Polymermaterial auf. Dieses Material kann ein Polymer sein, und kann biologisch abbaubar oder nicht biologisch abbaubar sein. Neben den oben aufgeführten Vorteilen bringt eine solche Ausgestaltung einige zusätzliche Vorteile mit sich.

Erstens besitzen Polymermaterialien in der Regel eine geringere Reißfestigkeit als metallische Materialien, die wie oben angedeutet vom Degradationsgrad des verwendeten Materials direkt abhängt. Bei der erfindungsgemäßen wachstumsfähigen Stützprothese wirkt sich eine erhöhte Reißfestigkeit an den Sollbruchstellen wachstumshemmend aus, da die Sollbruchstellen nur schwer auseinandergerissen werden können. Umgekehrt kann durch die Auswahl eines Materials mit erhöhter Reißtendenz an den Sollbruchstellen die Wachstumsfähigkeit der erfindungsgemäßen Stützprothese begünstigt werden. So hat man die Möglichkeit, durch Verwendung eines nicht metallischen Materials einer bekannten Reißfestigkeit an den Sollbruchstellen, Stützprothesen mit unterschiedlichen Wachstumsfähigkeiten bereitzustellen. So kann die Wachstumsfähigkeit der bereitgestellten Stützprothese den spezifischen klinischen Anforderungen eines jeden Patienten angepasst werden.

Zweitens wird durch die Ausgestaltung der Sollbruchstellen mit nicht metallischem Material gewährleistet, dass nach dem Aufbrechen des Maschengerüsts infolge des Wachstums oder des Expandierens keine scharfkantigen Ecken aus Metall gebildet werden, die später die abgestützte Gefäßwand verletzen oder gar abbrechen und einen entfernten Ort im Gefäßsystem beeinträchtigen könnten.

Für den Fall, dass das Maschengerüst der Stützprothese aus einem metallischem Material und die Sollbruchstellen aus einem nicht metallischen Material bestehen oder dieses umfassen, wird bevorzugt das nicht metallische Material in dem Bereich der Sollbruchstellen durch Beschichten auf das metallische Material der Stützprothese aufgebracht. Dabei weist das metallische Material des Maschengerüsts zumindest im Bereich der Sollbruchstellen, d. h. unterhalb der Beschichtung aus nicht metallischem Material, bevorzugt eine polierte Oberflächenstruktur bzw. eine abgerundete Form auf. So wird gewährleistet, dass auch nach dem Aufbrechen der Sollbruchstellen und gegebenenfalls nach dem Auflösen des nicht metallischen Materials eine glatte Oberfläche des Maschengerüsts bzw. keine scharfe Kante an diesen Sollbruchstellen zurückbleibt, wodurch der erwähnte Vorteil des verminderten Verletzungsrisikos realisiert wird. Verfahren zum Polieren von metallischen Feinstrukturen wie beispielsweise das Elektropolieren sind dem Fachmann bekannt.

Gemäß einer weiteren Ausführungsform der vorliegenden Erfindung weist ein Maschengerüst an jedem der zwei endständigen Bereiche der Stützprothese bezogen auf die Stützprothesenlängsachse mindestens zwei Gerüstringe auf, wobei die zwei dem Mittelpunkt der Stützprothesenlängsachse jeweils am nächsten angeordneten Gerüstringe über langgezogene Verbindungsglieder miteinander verbunden sind. So wird eine Stützprothese gebildet, die zwei jeweils endständig angeordnete Maschengerüstteile sowie einen mittleren Teil aufweist, der die zwei endständig angeordneten Maschengerüste über vorzugsweise lang gezogene Verbindungsglieder miteinander verbindet. Eine solche Stützprothese ist in solchen Fällen vorteilhaft, bei denen ein sich verzweigendes Gefäß langfristig abgestützt werden soll. So kann die Stützprothese gemäß dieser Ausführungsform derart ins abzustützende Gefäß eingeführt werden, dass das eine endständige Maschengerüst oberhalb der Verzweigungsstelle und das andere endständige Maschengerüst unterhalb der Verzweigungsstelle angeordnet ist, während in der Höhe der Verzweigungsstelle sich die im mittleren Teil der Stützprothese angeordneten, lang gezogenen Verbindungsglieder befinden. In dieser Weise wird eine Beschädigung oder eine Okklusion der Verzweigungsstelle vermieden, da der lang gezogene mittlere Teil der Stützprothese weniger robust ist und daher auf die Gefäßwände eine niedrigere radiale Kraft ausübt, als die endständig angeordneten Maschengerüste. So wird die empfindliche Gefäßstelle in bevorzugter Weise geschont. Das größere Lumen der Maschenöffnungen erlaubt außerdem einen ungehinderten Blutfluß in das abzweigende Gefäß. Im Laufe der Zeit können die beiden endständigen Maschengerüste, wie oben dargestellt, wachsen, wodurch eine langfristige unterstützende Wirkung der Gefäßabschnitte oberhalb und unterhalb der empfindlichen Gefäßstelle gewährleistet wird. Gemäß einer weiteren Ausführungsform der vorliegenden Erfindung beträgt die Länge der langgezogenen Verbindungsglieder das 0,75 bis 3-fache der oben erwähnten Amplitude A der Verformungen der Maschenringe. Bevorzugt beträgt die Länge der langgezogenen Verbindungsglieder das 1 bis 2-ache der oben erwähnten Amplitude A. Gemäß dieser Ausführungsform weist jedes endständige Maschengerüst 2-8, vorzugsweise 2-6, besonders bevorzugt 1-4, aber vorzugsweise nicht mehr als 10 Gerüstringe auf.

Die proximal und distal abstützenden Bereiche (d. h. das proximale und distale Maschengerüste können härter und steifer als die dazwischenliegenden langgezogenen Verbindungsglieder ausgestaltet sein. Sie können auch mit Verankerungsstrukturen versehen sein, um ein durch den Blutfluss verursachtes Abrutschen innerhalb des Gefäßes zu verhindern.

Gemäß einer weiteren Ausführungsform der vorliegenden Erfindung weist die Stützprothese an ihrer Innenseite eine Herzklappe auf. Dabei kann die Herzklappe an der Stützprothese genäht, geklebt, geklemmt oder gewebt werden, wobei es bevorzugt ist, die Herzklappe an der Stützprothese zu nähen. Hierbei kann es von Vorteil sein, die Herzklappe mittels eines Tissue-Engineering-Verfahrens herzustellen. Besonders vorteilhaft kann es sein, in diesem Tissue-Engineering-Verfahren Zellen derselben Herkunft wie bei dem Besiedeln des Maschengerüstes der Stützprothese zu verwenden, also homologe oder autologe Zellen. So kann gewährleistet werden, dass die jeweiligen Wachstumsgeschwindigkeiten der innerhalb der Stützprothese sich befindenden Herzklappe einerseits und das Maschengerüst der Stützprothese andererseits während des gesamten Wachstumsvorgangs im Wesentlichen gleich bleiben.

Die folgenden Abbildungen und Beispiele erläutern in nicht einschränkender Weise die Erfindung. Die Abbildungen zeigen:

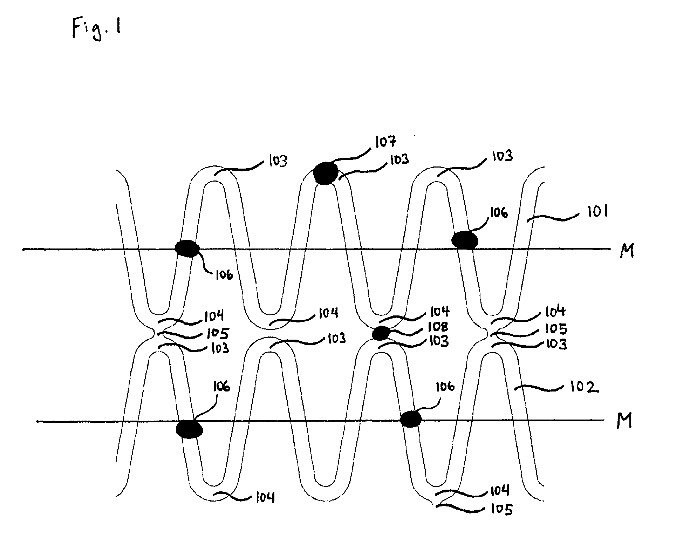

- Fig. 1:: Eine Draufsicht eines Abschnittes des Maschengerüstes einer wachstumsfähigen Stützprothese gemäß einer Ausführungsform der Erfindung. Dargestellt werden ein erster periodisch verformter Gerüstring (101) und ein zweiter peri-odisch verformter Gerüstring (102), jeweils mit Hochpunkten (103) und Tiefpunkten (104), die in deren Mitte die Umfangsmittellinie M definieren. Der erste und zweite Gerüstring (101 und 102) werden über als Streben ausgestaltete Verbindungsglieder (105) miteinander verbunden. Sollbruchstellen werden als schwarze Punkte dargestellt, die in der gezeigten Ausführungsform an oder neben der Mittellinie M (Sollbruchstellen 106), an einem Hochpunkt (Soll-bruchstelle 107) sowie in einem Verbindungsglied (Sollbruchstelle 108) ange-ordnet sind.
- Fig. 2A:: Einen Abschnitt (201) eines Gerüstringes oder Verbindungsglieds mit einer Sollbruchstelle, die als Perforation (202) ausgestaltet ist.
- Fig. 2B:: Einen Abschnitt (201) eines Gerüstringes oder Verbindungsglieds mit einer Sollbruchstelle, die mit oben und unten angeordneten, dreieckförmigen Kerben (203) ausgestaltet ist.
- Fig. 2C:: Einen Abschnitt (201) eines Gerüstringes oder Verbindungsglieds mit einer Sollbruchstelle, die mit oben angeordneter dreieckförmiger Kerbe (204) ausgestaltet ist.
- Fig. 2D:: Einen Abschnitt (201) eines Gerüstringes oder Verbindungsglieds mit einer Sollbruchstelle, die mit oben und unten angeordneten viereckförmigen Kerben (205) ausgestaltet ist.
- Fig. 2E:: Einen Abschnitt (201) eines Gerüstringes oder Verbindungsglieds mit einer Sollbruchstelle, die mit abgerundetem Schnitt ausgestaltet ist, und die mit einem Polymer (207) beschichtet ist.
- Fig. 2F:: Einen Abschnitt (201) eines Gerüstringes oder Verbindungsglieds mit einer Sollbruchstelle, die mit Stufenschnitt (208) ausgestaltet ist, und die mit einem Polymer (207) beschichtet ist.
- Fig. 2G:: Einen Querschnitt (211) eines Gerüstringes oder Verbindungsglieds mit einer Sollbruchstelle, die mit oben und unten angeordneten viereckförmigen Kerben (205) ausgestaltet ist.

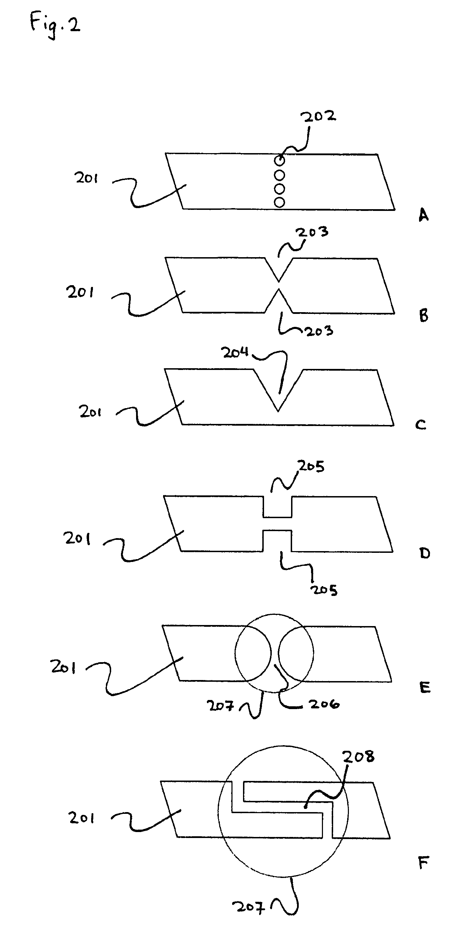

- Fig. 2F:: Einen Querschnitt (211) eines Gerüstringes oder Verbindungsglieds mit einer Sollbruchstelle, die mit oben, unten, links und rechts angeordneten viereckförmigen Kerben (205) ausgestaltet ist.

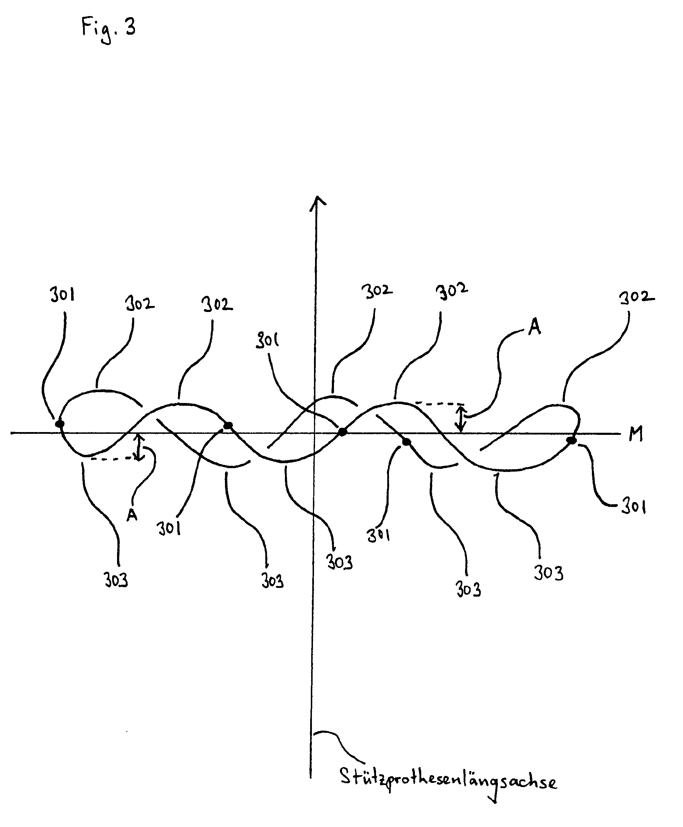

- Fig. 3: Eine Perspektivansicht eines Gerüstringes mit sinusförmigen Verformungen. Der gezeigte Gerüstring weist 5 Hochpunkte (302), 5 Tiefpunkte (303) und 5 Sollbruchstellen (301) an oder neben der senkrecht zur Stützprothesenlängsachse verlaufenden Mittellinie M auf. Ebenfalls gezeigt ist die Amplitude A. Nicht gezeigt sind Verbindungsglieder, die den dargestellten Gerüstring mit einem benachbarten Gerüstring im Maschengerüst verbinden.

Das nachfolgende Beispiel erläutert in nicht einschränkender Weise, wie eine wachstumsfähige Stützprothese hergestellt werden kann.

### Beispiel 1: Fertigung einer wachstumsfähigen Stützprothese

Im Allgemeinen umfasst die Produktion einer wachstumsfähigen Stützprothese folgende Verfahrensschritte:
1. Laserschneiden
2. Elektropolieren
3. Kontrollieren/Reinigen

### 1.1. Laserschneiden

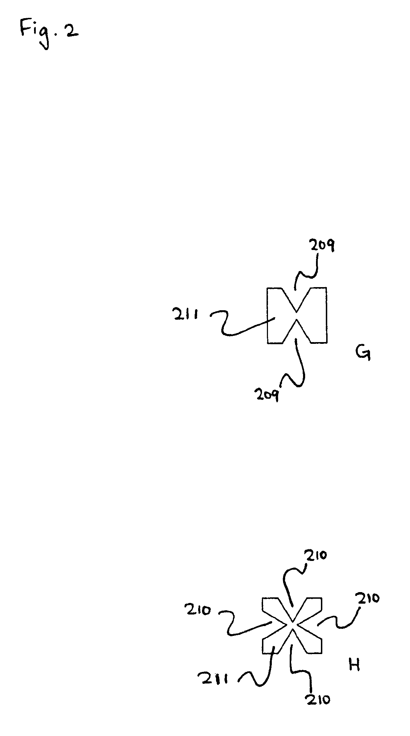

Die Stützprothese wird aus hochpräzisem Material (z. B. 316L, L605 oder Nitinol) unter Verwendung eines Festkörperlasers gefertigt. Um das Design der Stützprothese frei gestalten zu können, wird ein Verfahren angestrebt, das eine beliebige Schnittführung auf einem Rohr ermöglicht. Ein Laser erfüllt in Verbindung mit einem Koordinatentisch, einer Rundachse und einer speziell angefertigten Rohrführung, die das Rohr präzise in den erforderlichen Freiheitsgraden unter dem Laserschneidkopf führt, diese Anforderungen. Dabei werden die Stützprothesenstruktur und die in diesem Fall mechanischen Sollbruchstellen (wie beispielsweise in Fig.2 dargestellt) in einem Arbeitsgang in das Rohr geschnitten. Nach Entfernen der "Fenster", d.h. der ausgeschnittenen Bereiche bleiben die Streben der Stützprothese und damit die fertige Stützprothesenstruktur zurück. Dazu gehören sowohl die Gerüstringe als auch die diese verbindenden Verbindungsglieder.

### 1.2. Elektropolieren

Durch das dem Fachmann allgemein bekannte Elektropolieren werden die durch das Laserschneiden entstandenen Stützprothesenkanten zur Minimierung der Gefäß- und Implantationsballonverletzung abgerundet und die Stützprothesenoberflächen geglättet. Zum Einsatz kommt ein elektrochemisches Polierverfahren, das an die speziellen Gegebenheiten der Stützprothesenherstellung angepasst ist. Die Anpassung für die Stützprothesenbearbeitung umfasst hauptsächlich eine Poliervorrichtung, die einen gleichmäßigen Stromfluss während des Poliervorgangs gewährleistet. Dabei werden die Stützprothesen im Elektropolierbad kontinuierlich bewegt und gleichzeitig unter Spannung gesetzt.

### 1.3. Kontrollieren/Reinigen

Nach der Politur durch das oben beschriebene Elektropolierverfahren werden die Stützprothesen durch Dekapieren und mehrere Spülungen von Politurrückständen gereinigt und optisch kontrolliert sowie vermessen und verpackt. Bei der optischen Kontrolle wird die gesamte Oberfläche beurteilt . Dabei werden Stützprothesen aussortiert, die u. a. folgende Kriterien nicht erfüllen:
· gleichmäßige, glatte Oberfläche
· durchgängige Kantenverrundung ohne Spitzen

## Patentansprüche

1. Rohrförmige, wachstumsfähige Stützprothese, aufweisend ein Maschengerüst, wobei das Maschengerüst zumindest zwei miteinander über Verbindungsglieder verbundene, punktsymmetrisch um die Stützprothesenlängsachse angeordnete Gerüstringe aufweist, wobei die Gerüstringe und/oder die Verbindungsglieder zumindest eine Sollbruchstelle aufweisen.

2. Stützprothese nach Anspruch 1, wobei zumindest ein Gerüstring des Maschengerüsts n periodische, längs der Stützprothesenlängsachse sich erstreckende, Hoch- und Tiefpunkte bildende Verformungen aufweist, die eine sich auf die Stützprothesenlängsachse bezogene Amplitude A aufweisen, wobei n = 16-70, bevorzugt 20-56, besonders bevorzugt 24-42.

3. Stützprothese nach Anspruch 2, wobei die Verformungen sinusförmig, rechteckig, sägezahnförmig oder dreieckig ausgestaltet sind.

4. Stützprothese nach Anspruch 2 oder 3, wobei die Anzahl der periodischen Verformungen zweier jeweils benachbarter Gerüstringe identisch ist oder sich um ein ganzzähliges Vielfaches voneinander unterscheidet.

5. Stützprothese nach einem der Ansprüche 2 bis 4, wobei zwei benachbarte Gerüstringe derart phasenversetzt zueinander angeordnet sind, dass Hochpunkte eines Gerüstrings mit Tiefpunkten eines jeweils benachbarten Gerüstrings über die Verbindungsglieder verbunden sind.

6. Stützprothese nach einem der Ansprüche 1-5, wobei die Verbindungsglieder als Ringe, Klemmen oder sich parallel zur Stützprothesenlängsachse erstreckende Schlaufen, Fäden, Drähte oder Streben ausgestaltet sind.

7. Stützprothese nach einem der Ansprüche 1-6, wobei die Gerüstringe und/oder die Verbindungsglieder zumindest zum Teil aus einem biologisch abbaubaren Material bestehen.

8. Stützprothese nach Anspruch 7, wobei das biologisch abbaubare Material aus zumindest einer Legierung, zumindest einem Polymer oder zumindest einem Edelstahl mit Formgedächtnis ausgewählt ist.

9. Stützprothese nach Anspruch 8, wobei die Legierung mit Formgedächtnis eine Nickel-Titanlegierung, eine Aluminumlegierung, eine Magnesiumlegierung oder eine Eisenlegierung ist.

10. Stützprothese nach Anspruch 7, wobei das biologisch abbaubare Polymer Polyglykolsäure (PGA), Polymilchsäure (PLA), Polyhydroxyalkanoat (PHA), Poly-4-Hydroxybutyrat (P4HB), Polycaprolactone (PLGA), Polycarbonate, Polyamide, Polyanhydride, Polyaminosäuren, Polyorthoester, Polyacetate, Polycyanoacrylate sowie abbaubare Polyurethane und nicht erodierbare Polymere wie Polyacrylate, Ethylenvinylacetat-Polymere, andere substituierte Zelluloseacetate sowie Derivate davon, Polyester der Hydroxycarboxysäuren, Polyanhydride der Dicarboxyester, Copolymere der Hydroxycarboxysäuren und der Dicarboxyester, ein synthetisches Polymer aus mindestens einem Glykolid, Laktid, p-Dioxanon, Caprolacton, Trimethylencarbonat und/oder Butyrolacton, Polymere oder Copolymere von Glykolsäure, Milchsäure und Sebacinsäure, Polyhydroxyalkanoat-Zusammensetzungen von 2-, 3-, 4- oder 5-Hydroxysäuren, z. B. Poly-4-hydroxybutyrate, ein Poly-4-hydroxybutyrat-co-3-hydroxybutyrat, Homopolymere und Copolymere mit einer beliebigen Kombination von 3-Hydroxybutyrate, 3-Hydroxyvalerat, 3-Hydroxypropionat, 2-Hydroxybutyrat, 4-Hydroxybutyrat, 4-Hydroxyvalerat, 3-Hydroxyhexanoat, 3-Hydroxyheptanoat, 3-Hydroxyoctanoat, 3-Hydroxynonanoat, 3-Hydroxytridecanoat, 3-Hydroxytetradecanoat, 3-Hydroxypentadecanoat, 3-Hydroxyhexadecanoat, 3-Hydroxyheptadecanoat und 3-Hydroxyoctadecanoat oder eine Kombination davon ist.

11. Stützprothese nach einem der Ansprüche 2-10, wobei die zumindest eine Sollbruchstelle an oder neben dem Mittelpunkt zwischen benachbarten Hoch- und Tiefpunkten der periodischen Verformungen des Gerüstrings angeordnet ist.

12. Stützprothese nach einem der Ansprüche 1-11, aufweisend ein Maschengerüst an jedem der zwei endständigen Bereichen der Stützprothese bezogen auf die Stützprothesenlängsachse, wobei die zwei dem Mittelpunkt der Stützprothesenlängsachse am nächsten angeordneten Gerüstringe über langgezogene Verbindungsglieder miteinander verbunden sind.

13. Stützprothese nach Anspruch 12, wobei die Länge der langgezogenen Verbindungsglieder das 0,75-3-fache, bevorzugt das 1-2-fache der Amplitude A betragen.

14. Stützprothese nach einem der Ansprüche 1 bis 13, weiter aufweisend an ihrer Innenseite eine Herzklappe oder ein Gefäß.

15. Stützprothese nach Anspruch 14, wobei die Herzklappe an der Stützprothese genäht, geklebt, geklemmt oder gewebt ist.

16. Stützprothese nach einem der Ansprüche 14 oder 15, wobei die Herzklappe Produkt eines Tissue-Engineering Verfahrens ist.

17. Stützprothese nach einem der Ansprüche 1-16, wobei die zumindest eine Sollbruchstelle aus einem nicht metallischen Material bestehen.

18. Stützprothese nach einem der Ansprüche 1-17, wobei die zumindest eine Sollbruchstelle derart ausgestaltet ist, dass sie unter äußerer Einwirkung gesprengt, aufgelöst oder geschwächt werden kann.

19. Stützprothese nach Anspruch 18, wobei die äußere Einwirkung ausgewählt ist aus: Schallwellen; zumindest einem magnetischen Feld; einer Kombination aus magnetischem Feld und elektromagnetischem Feld; elektromagnetischer Strahlung; elektrischer Energie und einer beliebigen Kombination davon.

20. Stützprothese nach Anspruch 19, wobei die Schallwellen Ultraschall und/oder Stoßwellen sind.

21. Stützprothese nach Anspruch 19, wobei die Kombination aus magnetischem Feld und elektromagnetischem Feld Kernspinresonanz (Magnetic Resonanz Imaging, MRI) ist.

22. Stützprothese nach Anspruch 19, wobei die elektromagnetische Strahlung Röntgenstrahlung oder Infrarotstrahlung (thermische Energie) ist.

23. Stützprothese nach einem der Ansprüche 18-22, wobei die äußere Einwirkung von innerhalb oder außerhalb des Körpers erfolgt.

24. Stützprothese nach Anspruch 23, wobei die Einwirkung von innerhalb des Körpers mit Hilfe eines intravaskulären Katheters oder minimal invasiv mit Hilfe der Endoskopie erfolgt.

25. Stützprothese nach Anspruch 8, wobei das Polymer mit Formgedächtnis aus tert-Butylacrylat, Poly(ethylenglycol)dimethylacrylat oder PCL kombiniert mit 2,4-Toluoldiisocyanat ethylenglycol ausgewählt ist.
